(19) Europäisches Patentamt / European Patent Office / Office européen des brevets

(11) **EP 3 798 208 A1**

(12) **EUROPÄISCHE PATENTANMELDUNG**

(43) Veröffentlichungstag:
**31.03.2021 Patentblatt 2021/13**

(51) Int Cl.:
*C07C 263/20* (2006.01)   *C07C 265/14* (2006.01)
*B01J 3/00* (2006.01)   *F04C 19/00* (2006.01)

(21) Anmeldenummer: **19200482.8**

(22) Anmeldetag: **30.09.2019**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Covestro Deutschland AG**
**51373 Leverkusen (DE)**

(72) Erfinder: **Die Erfindernennung liegt noch nicht vor**

(74) Vertreter: **Levpat**
**c/o Covestro AG**
**Gebäude 4825**
**51365 Leverkusen (DE)**

(54) **VERFAHREN ZUR DESTILLATION VON ISOCYANATEN**

(57) Die Erfindung betrifft ein Verfahren zur destillativen Aufreinigung eines durch Phosgenierung des entsprechenden Amins erhaltenen Roh-Isocyanates in einer Destillationsapparatur im Vakuum, um das entsprechende Isocyanat zu erhalten, dadurch gekennzeichnet, dass mindestens ein Flüssigkeitsringverdichter eingesetzt wird, um das Vakuum in der Destillationsapparatur zu erzeugen, eine bestimmte Betriebsflüssigkeit für den mindestens einen Flüssigkeitsringverdichter verwendet wird, der Druck p auf der Ansaugseite des mindestens einen Flüssigkeitsringverdichters 10 bis 200 mbar(a) beträgt, die Betriebstemperatur des mindestens einen Flüssigkeitsringverdichters -17 bis +15 °C beträgt, und die Betriebsflüssigkeit am Austritt aus dem mindestens einen Flüssigkeitsringverdichter einen AC-Wert von weniger als 35000 ppm aufweist, eine entsprechende Verwendung mindestens eines Flüssigkeitsringverdichters zur Erzeugung eines Vakuums in einer Vorrichtung zur Destillation eines Roh-Isocyanates, und ein entsprechendes Verfahren zum Betreiben eines Flüssigkeitsringverdichters zum Bereitstellen des Vakuums für die destillative Aufreinigung eines durch Phosgenierung des entsprechenden Amins erhaltenen Isocyanates in einer Destillationsapparatur, um das entsprechende Isocyanat zu erhalten.

EP 3 798 208 A1

**Beschreibung**

Die Erfindung betrifft ein Verfahren zur destillativen Aufreinigung eines durch Phosgenierung des entsprechenden Amins erhaltenen Roh-Isocyanates in einer Destillationsapparatur im Vakuum, um das entsprechende Isocyanat zu erhalten, dadurch gekennzeichnet, dass mindestens ein Flüssigkeitsringverdichter eingesetzt wird, um das Vakuum in der Destillationsapparatur zu erzeugen, eine bestimmte Betriebsflüssigkeit für den mindestens einen Flüssigkeitsringverdichter verwendet wird, der Druck p auf der Ansaugseite des mindestens einen Flüssigkeitsringverdichters 10 bis 200 mbar(a) beträgt, die Betriebstemperatur des mindestens einen Flüssigkeitsringverdichters -17 bis +15 °C beträgt, und die Betriebsflüssigkeit am Austritt aus dem mindestens einen Flüssigkeitsringverdichter einen AC-Wert von weniger als 35000 ppm aufweist, eine entsprechende Verwendung mindestens eines Flüssigkeitsringverdichters zur Erzeugung eines Vakuums in einer Vorrichtung zur Destillation eines Roh-Isocyanates, und ein entsprechendes Verfahren zum Betreiben eines Flüssigkeitsringverdichters zum Bereitstellen des Vakuums für die destillative Aufreinigung eines durch Phosgenierung des entsprechenden Amins erhaltenen Isocyanates in einer Destillationsapparatur, um das entsprechende Isocyanat zu erhalten.

Erfindungsgemäß werden unter dem Begriff "Isocyanat" organische Verbindungen mit mindestens einer Isocyanat-Gruppe verstanden, die durch Phosgenierung des entsprechenden Amins erhalten wurden.

Im Stand der Technik werden verschiedene Verfahren zur Isolierung und Reinigung von Isocyanaten aus Phosgenierungs-Reaktionen beschrieben. In den gängigsten Verfahren wird die Rohlösung aus der Phosgenierung zunächst von überschüssigem Phosgen befreit. Anschließend wird das Lösungsmittel, meist Chlorbenzol oder Dichlorbenzol, in der sogenannten Lösungsmittelabtrennung destillativ entfernt, bevor das so gewonnene Roh-Isocyanat wiederum destillativ, meist in mehreren Stufen, aufgereinigt wird. Viele technisch relevante Isocyanate werden dabei verdampft und als Brüdenstrom über Kopf oder nach Kondensation als flüssiger Seitenstrom aus der Reindestillations-Kolonne entnommen.

Die Reindestillation verschiedener Isocyanate, die jeweils durch Phosgenierung der entsprechenden Amine erhalten wurden, ist beispielsweise in US 3,549,504 beschrieben. Dort erfolgt die Destillation in Gegenwart eines Inertgases und bei einem Druck am Kopf der Kolonne von 5 bis 200 mmHg, vorzugsweise 20 bis 150 mmHg, so dass die Temperatur am Sumpf der Kolonne möglichst gering gehalten werden kann, um Zersetzungsreaktionen zu vermeiden. Die Ausführungsbeispiele nennen für die Destillation von Xylylendiisocyanat (XDI) bzw. Toluylentriisocyanat jeweils einen Kopfdruck von 30 mmHg, für Diphenylmethandiisocyanat (MDI) einen Kopfdruck von 20 mmHg, für Hexamethylendiisocyanat (HDI) einen Kopfdruck von 50 mmHg und für Toluylendiisocyanat (TDI) einen Kopfdruck von 40 mmHg.

In der WO2017/076551 A1 wird eine Trennwandkolonne beschrieben, die sich für die Destillation von Isocyanaten wie beispielsweise TDI, MDI, HDI, Pentamethylendiisocyanat (PDI), Isophorondiisocyanat (IPDI), XDI oder Dicyclohexylmethandiisocyanat ($H_{12}$MDI) eignet, wobei das gewünschte Isocyanat jeweils als Seitenstrom entnommen wird, während leichtsiedende Komponenten am Kopf der Kolonne anfallen. Als Ausführungsbeispiel wird die Destillation eines Roh-TDI-Gemisches bei einem Kopfdruck von 70 mbar beschrieben.

Eine weitere Beschreibung eines Verfahrens zur Reinigung von Isocyanaten findet sich in der DE 10260092 A1. Hier wird beschrieben, dass nach der Herstellung des Isocyanates zunächst Chlorwasserstoff, Phosgen und Lösungsmittel abgetrennt werden, um einen Roh-Isocyanat-Strom zu erhalten. Dieser enthält noch Komponenten mit einem niedrigeren Siedepunkt als das Isocyanat, Komponenten mit einem höheren Siedepunkt als das Isocyanat sowie einen nicht verdampfbaren Rückstand. Das Roh-Isocyanat wird im Vakuum bei 1 bis 120 mbar(a) grob-, und anschließend bei 1 bis 80 mbar(a) in mindestens einer Kolonne feindestilliert. Aus dem in der Grobdestillation erhaltenen Rückstand wird bei einem Druck von 0,1 bis 40 mbar(a) weiteres Isocyanat dampfförmig zurückgewonnen und ebenfalls der Feindestillation zugeführt. Als Ausführungsbeispiel ist eine Destillation von TDI beschrieben, bei der die Kolonne einen Kopfdruck von 18 mbar(a) aufweist.

In der DE 2242626 A1 wird ein Verfahren zum Eindampfen phosgenhaltiger Lösungen beschrieben, bei dem ein organisches Lösungsmittel von Verbindungen enthaltend Stickstoff oder Sauerstoff mit einem höheren Siedepunkt, beispielsweise Isocyanate, im Vakuum abgetrennt wird. Das Vakuum wird mit einer Flüssigkeitsringpumpe erzeugt, welche als Betriebsflüssigkeit ein phosgenhaltiges Lösungsmittel verwendet. In dem beschriebenen Verfahren wird ein Lösungsmittel von einem Isocyanat in einem Verdampfer abgetrennt, wobei das Isocyanat als Konzentrat im Sumpf des Verdampfers anfällt. Es handelt sich hierbei somit um die eingangs erwähnte Lösungsmittelabtrennung als Teil der Aufarbeitung eines Rohprodukts aus der Phosgenierung eines Amins. Als Druckbereiche für den Betrieb des Verdampfers werden 5 bis 2000 Torr, vorzugsweise 50 bis 500 Torr, genannt, im Ausführungsbeispiel wird ein Druck von 160 Torr genannt. Es wird darauf hingewiesen, dass es möglich ist, einen Teilstrom der Betriebsflüssigkeit über einen Wärmetauscher im Kreis zu pumpen, um die durch den Antrieb und die Absorption von Phosgen entstandene Wärme abzuführen. Des Weiteren wird darauf hingewiesen, dass sich Phosgen in der Betriebsflüssigkeit anreichert und den Wirkungsgrad der Pumpe verringern kann, weshalb ein fortlaufender Austausch erfolgen muss. Gleichzeitig ist die Absorption von Phosgen in dem Betriebsmittel als Vorteil des Verfahrens beschrieben, da auf diese Weise das Abgas von Phosgen befreit wird. Es wird in dem Dokument auch auf die Möglichkeit hingewiesen, das beschriebene Verfahren in der Enddestillation, also in der eingangs erwähnten Destillation des Isocyanat-Konzentrats, einzusetzen, da in dieser Destillation noch gebundenes Phosgen abgespalten und freigesetzt werden kann.

In der Praxis kommt es bei der Verwendung von Flüssigkeitsringverdichtern in der Reindestillation von durch Phosgenierung von Aminen hergestellten Isocyanaten jedoch überraschenderweise immer wieder zu unterschiedlich stark ausgeprägter Kavitation, die sich in Form von Geräuschen bis hin zur mechanischen Beschädigung des Verdichters äußert. Eine Absenkung der Betriebstemperatur könnte dem Problem entgegen wirken, ist jedoch einerseits nur mit erheblichem apparativem und energetischem Aufwand zu erreichen und andererseits spätestens durch die Erstarrungstemperatur der jeweiligen Betriebsflüssigkeit begrenzt. Das Problem der Kavitation tritt insbesondere bei Isocyanaten auf, die eine geringere Stabilität aufweisen und deshalb möglichst schonend destilliert werden müssen, deren Destillation also, wie aus dem zuvor zitierten Stand der Technik bekannt, einen möglichst niedrigen Druck erfordert, um Oligomerisierungs- und Zersetzungsreaktionen weitgehend zu vermeiden.

Um Kavitation in Flüssigkeitsring-Verdichtern zu vermeiden, wurden in der Vergangenheit bereits verschiedene Ansätze erprobt. So ist beispielsweise in der DE 10019718 A1 beschrieben, das angesaugte Gas vor Eintritt in den Flüssigkeitsringverdichter mittels eines Kühlgases, vorzugsweise Umgebungsluft, zu kühlen. Diese Vorgehensweise ist vorteilhaft für das Ansaugen heißer Dämpfe bei hohem Saugdruck, jedoch ist sie für den vorliegenden Fall der Anwendung in der Isocyanat-Destillation ungeeignet, da die Maßnahme für die erforderlichen niedrigen Saugdrücke nicht ausreichend ist. Die erforderliche Betriebstemperatur des Flüssigkeitsringverdichters liegt regelmäßig unterhalb der Umgebungstemperatur und auch das Vorhandensein von leicht flüchtigen Substanzen wie beispielsweise Phosgen erschwert die Problematik der Kavitation.

Das Dokument WO 2006/029884 A1 beschreibt ein Verfahren zum Betrieb eines Flüssigkeitsringverdichters, bei dem als Betriebsflüssigkeit eine ionische Flüssigkeit verwendet wird. Auch diese Vorgehensweise ist mit verschiedenen Nachteilen behaftet. Zunächst wird mit der ionischen Flüssigkeit ein bislang nicht benötigter, meist teurer, Stoff in das Verfahren eingebracht, der beschafft, gelagert, gefördert und entsorgt werden muss. Darüber hinaus hat zwar die ionische Flüssigkeit selbst keinen Dampfdruck, jedoch ist nicht auszuschließen, dass sich flüchtige Bestandteile der angesaugten Flüssigkeit in ihr anreichern. Auch unter der Annahme, dass sich keine flüchtigen Substanzen anreichern, hätte das Verfahren den Nachteil, dass der erwünschte Effekt, Phosgen aus dem angesaugten Gas zu absorbieren, entfällt. Aufgrund der in ionischen Flüssigkeiten vorhandenen funktionellen Gruppen und der hohen Reaktivität von beispielsweise Phosgen ist letztlich auch nicht auszuschließen, dass es zu unerwünschten Reaktionen zwischen der ionischen Flüssigkeit und Bestandteilen der angesaugten Flüssigkeit kommt.

Ausgehend von diesem Stand der Technik lag der vorliegenden Erfindung die Aufgabe zu Grunde, Isocyanate unter Verwendung von Flüssigkeitsringverdichtern zur Vakuumerzeugung zu destillieren, bei dem auch bei einem geringem Saugdruck, insbesondere unterhalb von 200 mbar(a) Kavitation in der Betriebsflüssigkeit vermieden wird. Des Weiteren soll erfindungsgemäß ein Verfahren zur Erzeugung eines Vakuums bereitgestellt werden, bei dem ein ohnehin im Herstellungsprozess verwendetes Lösungsmittel als Betriebsflüssigkeit eingesetzt werden kann, bei dem das Abgas von Phosgen und/oder Chlorwasserstoff gereinigt werden kann und bei dem es unproblematisch ist, wenn eine Flüssigkeit in der Form von Tröpfchen im Ansaugstrom vorliegen kann.

Diese Aufgaben werden erfindungsgemäß gelöst durch das Verfahren zur destillativen Aufreinigung eines durch Phosgenierung des entsprechenden Amins erhaltenen Roh-Isocyanates in einer Destillationsapparatur im Vakuum, um das entsprechende Isocyanat zu erhalten, wobei

- mindestens ein Flüssigkeitsringverdichter eingesetzt wird, um das Vakuum in der Destillationsapparatur zu erzeugen,
- eine Betriebsflüssigkeit ausgewählt aus der Gruppe bestehend aus Chlorbenzol, Dichlorbenzol, Xylol, 1-Chlor-2,4-dimethylbenzol, Chlortoluol und Mischungen davon für den mindestens einen Flüssigkeitsringverdichter verwendet wird,
- der Druck p auf der Ansaugseite des mindestens einen Flüssigkeitsringverdichters 10 bis 200 mbar(a) beträgt,
- die Betriebstemperatur des mindestens einen Flüssigkeitsringverdichters -17 bis +15 °C beträgt, und
- die Betriebsflüssigkeit am Austritt aus dem mindestens einen Flüssigkeitsringverdichter einen AC-Wert von weniger als 35000 ppm aufweist.

Des Weiteren werden die Aufgaben erfindungsgemäß gelöst durch Verwendung mindestens eines Flüssigkeitsringverdichters zur Erzeugung eines Vakuums in einer Vorrichtung zur Destillation eines Roh-Isocyanates, das durch Phosgenierung des entsprechenden Amins hergestellt wurde, wobei

- eine Betriebsflüssigkeit ausgewählt aus der Gruppe bestehend aus Chlorbenzol, Dichlorbenzol, Xylol, 1-Chlor-2,4-dimethylbenzol, Chlortoluol und Mischungen davon für den mindestens einen Flüssigkeitsringverdichter verwendet wird,
- der Druck p auf der Ansaugseite des mindestens einen Flüssigkeitsringverdichters 10 bis 200 mbar(a) beträgt,
- die Betriebstemperatur des mindestens einen Flüssigkeitsringverdichters -17 bis +15 °C beträgt, und

- die Betriebsflüssigkeit am Austritt aus dem mindestens einen Flüssigkeitsringverdichter einen AC-Wert von weniger als 35000 ppm aufweist.

Bevorzugt betrifft die vorliegende Erfindung die erfindungsgemäße Verwendung, wobei in der Betriebsflüssigkeit am Austritt aus dem Flüssigkeitsringverdichter der Gehalt an Phosgen $c(COCl_2)$ 0,001 bis 4,5 Gew.-% beträgt, und der Gehalt an Chlorwasserstoff $c(HCl)$ von weniger als 1,6 Gew.-% beträgt.

Schließlich werden die Aufgaben erfindungsgemäß gelöst durch ein Verfahren zum Betreiben eines Flüssigkeitsringverdichters zum Bereitstellen des Vakuums für die destillative Aufreinigung eines durch Phosgenierung des entsprechenden Amins erhaltenen Isocyanates in einer Destillationsapparatur, um das entsprechende Isocyanat zu erhalten, wobei

- eine Betriebsflüssigkeit ausgewählt aus der Gruppe bestehend aus Chlorbenzol, Dichlorbenzol, Xylol, 1-Chlor-2,4-dimethylbenzol, Chlortoluol und Mischungen davon für den mindestens einen Flüssigkeitsringverdichter verwendet wird,
- der Druck p auf der Ansaugseite des mindestens einen Flüssigkeitsringverdichters 10 bis 200 mbar(a) beträgt,
- die Betriebstemperatur des mindestens einen Flüssigkeitsringverdichters -17 bis +15 °C beträgt, und
- die Betriebsflüssigkeit am Austritt aus dem mindestens einen Flüssigkeitsringverdichter einen AC-Wert von weniger als 35000 ppm aufweist.

Die vorliegende Erfindung wird im Folgenden detailliert beschrieben. Für das erfindungsgemäße Verfahren zur destillativen Aufreinigung eines durch Phosgenierung des entsprechenden Amins erhaltenen Roh-Isocyanates, für die erfindungsgemäße Verwendung mindestens eines Flüssigkeitsringverdichters zur Erzeugung eines Vakuums in einer Vorrichtung zur Destillation eines Roh-Isocyanates und für das erfindungsgemäße Verfahren zum Betreiben eines Flüssigkeitsringverdichters zum Bereitstellen des Vakuums für die destillative Aufreinigung eines durch Phosgenierung des entsprechenden Amins erhaltenen Isocyanates in einer Destillationsapparatur gelten die im Folgenden gemachten Ausführungen, Details und bevorzugten Ausführungsformen entsprechend.

Im Rahmen der vorliegenden Erfindung wird mindestens ein Flüssigkeitsringverdichter verwendet. Es ist erfindungsgemäß möglich, dass in einer entsprechenden Vorrichtung, insbesondere einer entsprechenden Destillationsapparatur ein oder mehrere, beispielsweise 2 bis 8, Flüssigkeitsringverdichter eingesetzt werden, wobei, wenn mehr als ein Flüssigkeitsringverdichter vorliegen, einer, einige oder alle der vorhandenen Flüssigkeitsringverdichter, gemäß der vorliegenden Erfindung betrieben werden können.

Der Aufbau und das Wirkprinzip eines Flüssigkeitsringverdichters sind dem Fachmann im Allgemeinen bekannt. Die Funktion beruht auf einem exzentrisch in einem teilweise mit einer Betriebsflüssigkeit gefüllten, meist zylindrischen, Gehäuse angeordneten Laufrad mit daran angesetzten Flügeln. Im Betrieb rotiert das Laufrad und die im Gehäuse enthaltene Betriebsflüssigkeit bildet durch die Zentrifugalkraft einen Flüssigkeitsring aus. Dadurch entstehen zwischen den Flügeln gasgefüllte Kammern, die auf der Außenseite durch den Flüssigkeitsring begrenzt und wegen der Exzentrizität unterschiedlich groß sind. Durch die Rotation des Laufrades ändert sich ständig die Größe der einzelnen Kammern, so dass auf der Saugseite durch eine oder mehrere Saugöffnungen Gas aus dem Saugstutzen angesaugt wird, dieses dann komprimiert wird und schließlich auf der Druckseite durch eine oder mehrere Drucköffnungen in Richtung Druckstutzen ausgestoßen wird. Mit dem Gas wird stets auch ein Teil der den Ring bildenden Betriebsflüssigkeit ausgestoßen. Diese kann in einem Flüssigkeitsabscheider vom Gas getrennt werden und wird im Rahmen der vorliegenden Erfindung als "Betriebsflüssigkeit am Austritt aus dem Flüssigkeitsringverdichter" bezeichnet. Sie kann gegebenenfalls nach Kühlung und/oder Beimischung weiterer ausreichend kühler Flüssigkeit wieder als Betriebsflüssigkeit verwendet werden. In einigen Bauformen von Flüssigkeitsringverdichtern gibt es einen zusätzlichen Entnahmestutzen für Betriebsflüssigkeit, beispielsweise, um einen größeren Kühlkreislauf aufzubauen. Aufgrund der starken Durchmischung im Verdichter entspricht die hier entnommene Flüssigkeit der am Druckstutzen mit dem Gas austretenden Betriebsflüssigkeit.

Eine Sonderform der Flüssigkeitsringverdichter stellen die Seitenkanalpumpen dar, bei denen das Laufrad konzentrisch angeordnet ist und der Pumpeffekt durch seitlich von diesem vorhandene kanalartige Räume wechselnder Tiefe erzielt wird. Diese werden jedoch nur selten als Vakuumpumpen verwendet.

Isocyanate bzw. Roh-Isocyanate, die sich mit dem erfindungsgemäßen Verfahren reinigen lassen sind im Allgemeinen alle dem Fachmann bekannten aliphatischen, cycloaliphatischen, araliphatischen oder aromatischen Isocyanate.

Besonders bevorzugt betrifft die vorliegende Erfindung das erfindungsgemäße Verfahren, wobei das Isocyanat ausgewählt ist aus der Gruppe bestehend aus Monoisocyanaten, insbesondere R,S-1-Phenylethylisocyanat, 1-Methyl-3-Phenylpropylisocyanat, Pentylisocyanat, 6-Methyl-2-heptanisocyanat, Cyclopentylisocyanat oder 3-(Methylthio)phenylisocyanat, Diisocyanaten, insbesondere Hexamethylendiisocyanat (HDI), Pentamethylendiisocyanat (PDI), 1,4-Butandiisocyanat, 1,8-Diisocyanatooctan, 1,9-Diisocyanatononan, 2-Methylpentamethylendiisocyanat, 2,2-Dimethylpentamethylendiisocyanat, Neopentandiisocyanat, 2,2,4-Trimethylhexamethylendiisocyanat und 2,4,4-Trimethylhexamethylendiisocyanat, 3-Isocyanatmethyl-3,5,5-trimethylcyclohexylisocyanat (IPDI), 2,4' und 4,4'-Diisocyanatodicyclohexylmethan ($H_{12}$MDI), 4,4'-Diisocyanato-3,3'-dimethyldicyclohexylmethan, 2,4- und 2,6-Diisocyanato-methyl-cyclohexan ($H_6$TDI), 1,3-Cyclohexandiisocyanat, 1,4-Cyclohexandiisocyanat, 1,3-Diisocyanato-2-methylcyclohexan, 1,3-Diisocyanato-4-methyl-cyclohexan, 1,4-Diisocyanato-3,3,5-trimethylcyclohexan, 1,3-Bis(isocyanatomethyl)-cyclohexan ($H_6$XDI), 1,4-Bis(isocyanatomethyl)cyclohexan, die Isomere des Bis(isocyanatomethyl)bicyclo[2.2.1]heptans (NBDI), Xylylendiisocyanat (XDI), insbesondere 1,3-Xylylendiisocyanat (m-XDI) oder 1,4-Xylylendiisocyanat (p-XDI), 1,3-Bis(1-isocyanato-1-methylethyl)benzol (m-TMXDI), 1,4-Bis(1-isocyanato-1-methylethyl)benzol (p-TMXDI), monomeres Diphenylmethandiisocyanat, 2,4-Toluylendiisocyanat, 2,6- Toluylendiisocyanat, 1,5-Naphthalindiisocyanat oder 1,4-Phenylendiisocyanat, Triisocyanaten, insbesondere 4-Isocyanatomethyl-1,8-octandiisocyanat, und Mischungen davon.

Besonders bevorzugt wird das erfindungsgemäße Verfahren zur Reinigung eines Isocyanates ausgewählt aus der Gruppe bestehend aus HDI, PDI, IPDI, H12MDI, XDI, H6-XDI, H6-TDI, MDI, NDI und Mischungen eingesetzt. Ganz besonders bevorzugt wird das Verfahren zur Reinigung von HDI, XDI, PDI oder TDI eingesetzt. Gerade PDI und XDI, vor allem PDI, fallen in der Phosgenierung mit hohen Anteilen an chlorierten Nebenprodukten an, aus denen unter thermischer Belastung leichtflüchtige Verbindungen wie Phosgen oder Chlorwasserstoff abgespalten werden können, so dass das erfindungsgemäße Verfahren insbesondere für die Destillation von PDI und XDI, am meisten für die Destillation von PDI, Vorteile bietet.

Verfahren zur Herstellung der erfindungsgemäß eingesetzten Isocyanate sind dem Fachmann an sich bekannt. Organische Isocyanate, die durch Phosgenierung der entsprechenden Amine hergestellt werden, enthalten stets verschiedene leicht- und schwersiedende, teilweise chlorhaltige Nebenprodukte. Die Begriffe "leichtsiedend" und "schwersiedend" sind dabei im Verhältnis zum Isocyanat zu sehen. Es ist für das erfindungsgemäße Verfahren nicht wesentlich, ob die Phosgenierung in der Gas- oder in der Flüssigphase erfolgt, und ob das Amin direkt mit Phosgen umgesetzt wird, beispielsweise in einer dem Fachmann bekannten Basen-Phosgenierung, oder ob es zunächst mit einer sauren Verbindung wie Chlorwasserstoff, wie in der dem Fachmann bekannten Hydrochlorid-Phosgenierung, oder Kohlendioxid, wie in der der Fachmann bekannten Carbaminat-Phosgenierung, in ein Salz überführt und dann mit Phosgen umgesetzt wird. Für alle Varianten sind technische Verfahren hinlänglich bekannt und sollen im Folgenden kurz umrissen werden.

Die vorliegende Erfindung betrifft daher das erfindungsgemäße Verfahren, wobei das Roh-Isocyanat aus einer Gasphasen-Phosgenierung oder einer Flüssigphasen-Phosgenierung stammt.

Erfolgt die Phosgenierung in der Gasphase, so wird das Amin bevorzugt verdampft und auf eine Temperatur innerhalb des Temperaturbereichs von 200 bis 600 °C erhitzt. Optional erfolgen die Verdampfung und auch der Einsatz der bei der Verdampfung erzeugten Amin-Dämpfe in Gegenwart eines Inertgases und/oder von Dämpfen eines inerten Lösungsmittels. Bevorzugtes Inertgas ist Stickstoff. Geeignete inerte Lösungsmittel sind beispielsweise Chlorbenzol, Dichlorbenzol, insbesondere o-Dichlorbenzol, Xylol, 1-Chlor-2,4-dimethylbenzol, Chlortoluol, insbesondere o-Chlortoluol, oder deren Gemische. Technisch haben sich insbesondere Chlorbenzol und o-Dichlorbenzol oder Mischungen davon durchgesetzt und sind zu bevorzugen, da diese sich unter den Reaktionsbedingungen einerseits sehr inert verhalten und andererseits aufgrund der physikalischen Eigenschaften, insbesondere aufgrund ihres Siedepunkts, in der Regel einfach vom Isocyanat abzutrennen sind.

Das bei der Phosgenierung verwendete Phosgen wird, bezogen auf das Amin, bevorzugt im Überschuss eingesetzt. Im Allgemeinen genügt eine Phosgenmenge, die 150 bis 350% der Theorie bezüglich der ablaufenden Phosgenierungsreaktion entspricht. Der Phosgenstrom wird vor der Reaktion bevorzugt auf eine Temperatur innerhalb des Bereichs von 200 bis 600 °C erhitzt.

Zur Durchführung der Phosgenierung werden der vorerhitzte, aminhaltige Strom und der ebenfalls vorerhitzte Phosgenstrom bevorzugt kontinuierlich in einen zylindrischen Reaktionsraum geleitet und dort miteinander vermischt. Geeignete zylindrische Reaktionsräume sind beispielsweise Rohrreaktoren, welche im Allgemeinen aus Stahl, Glas, legiertem und/oder emailliertem Stahl bestehen. Sie weisen im Allgemeinen eine Länge auf, die ausreichend ist, um unter den Verfahrensbedingungen eine vollständige Umsetzung des Amins mit dem Phosgen zu ermöglichen. Des Weiteren sind die Abmessungen des Reaktionsraums bevorzugt so gewählt, dass im Reaktionsraum eine turbulente Strömung mit einer Reynoldszahl von mindestens 2500 vorherrscht. Dies ist im Allgemeinen dann gewährleistet, wenn die Strömungsgeschwindigkeit mehr als 90 m/s beträgt. Eine solche Strömungsgeschwindigkeit kann durch Einstellen eines entsprechenden Differenzdrucks zwischen den Produktleitungen zum Reaktionsraum und dem Ausgang aus dem Reaktionsraum sichergestellt werden. Im Allgemeinen liegt der Druck in den Zuleitungen bei 200 bis 300 mbar(ü) und am Ausgang aus dem Reaktionsraum bei 150 bis 200 mbar(ü).

Nach der erfolgten Phosgenierungsreaktion im Reaktionsraum wird das den Reaktionsraum kontinuierlich verlassende Gemisch bevorzugt von dem gebildeten Isocyanat befreit. Dies kann beispielsweise durch selektive Kondensation in einem inerten Lösungsmittel wie beispielswiese Chlorbenzol oder Dichlorbenzol erfolgen. Sofern der aminhaltige Strom bereits ein inertes Lösungsmittel enthielt, ist es bevorzugt, hier das gleiche inerte Lösungsmittel zu verwenden. Die Temperatur des Lösungsmittels wird dabei bevorzugt so gewählt, dass sie einerseits oberhalb der Zersetzungstemperatur des dem Isocyanat entsprechenden Carbamidsäurechlorids liegt und anderseits das Isocyanat kondensiert bzw. sich in dem Lösungsmittel löst, während Phosgen, Chlorwasserstoff und gegebenenfalls Inertgas die Kondensationsstufe gasförmig durchlaufen. Besonders geeignet sind Lösungsmitteltemperaturen von 120 bis 200 °C.

Das die Kondensationsstufe zur Gewinnung des mindestens einen Isocyanates durchlaufende Gasgemisch wird anschließend bevorzugt in an sich bekannter Weise von überschüssigem Phosgen befreit. Dies kann mittels einer Kühlfalle, Absorption in einem inerten Lösungsmittel (z.B. Chlorbenzol, MCB, oder Dichlorbenzol, ODB) bei einer Temperatur von -10 °C bis 8 °C oder durch Adsorption und Hydrolyse an Aktivkohle erfolgen. Das die Phosgen-Rückgewinnungsstufe durchlaufende Chlorwasserstoffgas kann bevorzugt in an sich bekannter Weise zur Rückgewinnung des zur Phosgensynthese erforderlichen Chlors recykliert werden.

Eine andere Ausführungsform der Phosgenierung ist die Phosgenierung von Aminen in der flüssigen Phase. Die Reaktion kann dann auf verschiedene, dem Fachmann jeweils bekannte, Weisen ausgeführt werden. Entweder wird das Amin direkt mit Phosgen in einem inerten flüssigen Medium umgesetzt, so genannte Basenphosgenierung, oder das Amin wird zunächst durch Umsetzung mit Chlorwasserstoffgas oder Kohlendioxid in einem inerten flüssigen Medium in das entsprechende Salz überführt und dann mit Phosgen umgesetzt, so genannte Hydrochlorid- bzw. Carbaminatphosgenierung. Als flüssiges Medium eigenen sich für alle Phosgenierungen insbesondere Chlorbenzol und/oder Dichlorbenzol.

Bei der Basenphosgenierung wird die Reaktion zweistufig in dem inerten flüssigen Medium durchgeführt. Solche Reaktionen sind beispielsweise in W. Siefken, Liebigs Annalen der Chemie, 562 (1949) S. 96 beschrieben. Im ersten Stadium, der Kalt-Phosgenierung, wird die Temperatur der Reaktionsmischung bevorzugt in einem Bereich zwischen 0 und 100 °C gehalten. Es entsteht eine Suspension, die Carbaminsäurechlorid, Aminhydrochlorid und geringe Mengen an freiem Isocyanat enthält. Bevorzugt wird dabei eine Lösung von Phosgen in einem inerten Lösungsmittel vorgelegt und dann eine Lösung oder Suspension des Amins im gleichen Lösungsmittel sowie gegebenenfalls weiteres Phosgen zugegeben. Auf diese Weise wird die Konzentration von freiem Amin gering gehalten und somit die unerwünschte Bildung von Harnstoffen unterdrückt.

Im zweiten Stadium, der Heiß-Phosgenierung, wird die Temperatur erhöht und liegt vorzugsweise in einem Bereich von 120 bis 200 °C. Sie wird in diesem Bereich gehalten, während so lange weiteres Phosgen zugeführt wird, bis die Umsetzung zum Isocyanat beendet ist, also die HCl-Entwicklung zum Erliegen kommt. Phosgen wird dabei zweckmäßig im Überschuss verwendet. Bei Bedarf kann die Reaktion sowohl in der Kalt- als auch in der Heißphosgenierung unter Einleitung eines inerten Gases durchgeführt werden.

Bei der Hydrochlorid- oder Carbaminat-Phosgenierung wird das Amin bevorzugt zunächst mit Chlorwasserstoffgas oder Kohlendioxid in einem inerten flüssigen Medium zur Erzeugung des entsprechenden Salzes umgesetzt. Die Reaktionstemperatur liegt während dieser Salzbildung vorzugsweise in einem Bereich von 0 bis 80 °C. Es schließt sich der Phosgenierschritt als zweiter Schritt an, der im Wesentlichen der Heiß-Phosgenierung aus der oben beschriebenen Basenphosgenierung ähnelt. Es wird also auch hier die Temperatur bevorzugt im Bereich von 120 bis 200 °C gehalten, während Phosgen und optional ein Inertgas in die Reaktionsmischung eingeleitet werden. Die Einleitung erfolgt so lange, bis die Umsetzung zum Isocyanat beendet ist. Auch hier wird Phosgen vorzugsweise im Überschuss eingesetzt, um die Reaktion zu beschleunigen.

Sowohl bei der Basenphosgenierung als auch bei der Hydrochlorid- bzw. der Carbaminat-Phosgenierung wird nach Beendigung der Reaktion das verbliebene Phosgen und Chlorwasserstoffgas bevorzugt mit einem Inertgas, vorzugsweise mit Stickstoff, ausgeblasen. Bei Bedarf kann eine Filtration erfolgen, um gegebenenfalls vorhandene Feststoffe wie beispielsweise nicht weiter umgesetztes Hydrochlorid zu entfernen.

Das zunächst bei einer solchen Phosgenierung anfallende Reaktionsprodukt enthält neben dem gewünschten Isocyanat noch Reste an Chlorwasserstoff, Phosgen, Lösungsmittel sowie Verunreinigungen und nicht verdampfbaren Rückstand. Bei dem erfindungsgemäß zu reinigenden Roh-Isocyanat handelt es sich bevorzugt um ein solches Reaktionsprodukt, aus dem bereits in einem oder mehreren Schritten, bevorzugt durch Destillation, Phosgen und Chlorwasserstoff abgetrennt wurden.

Besonders bevorzugt handelt es sich bei dem erfindungsgemäß zu reinigenden Roh-Isocyanat um ein solches Roh-Isocyanat, bei dem auch die Hauptmenge an Lösungsmittel bereits abgetrennt wurde. Bevorzugt enthält das Roh-Isocyanat entsprechend nur noch Spuren an Phosgen und Chlorwasserstoff sowie eine geringe Menge Lösungsmittel. Darüber hinaus enthält es andere leichtsiedende Nebenkomponenten sowie schwersiedende Nebenkomponenten und nicht verdampfbaren Rückstand. Zumindest bei einem Teil der Nebenkomponenten und/oder des nicht verdampfbaren Rückstands handelt es sich bevorzugt um chlorhaltige Verbindungen.

Der Gehalt an hydrolysierbarem Chlor (HC-Wert) in Isocyanaten kann gemäß der ISO 15028:2014 bestimmt werden und liegt für das erfindungsgemäß eingesetzte Roh-Isocyanat vorzugsweise bei 200 bis 20000 ppm.

Erfindungsgemäß wird das Roh-Isocyanat im Vakuum destilliert, um Ausbeuteverluste aufgrund von Oligomerenbildung und/oder Zersetzung des Isocyanates zu minimieren. Die Destillation kann im Grunde in jeder geeigneten, dem Fachmann bekannten Destillationsvorrichtung erfolgen. Diese umfasst im Allgemeinen mindestens einen Verdampfer zum Verdampfen mindestens eines Teils des Roh-Isocyanates und einen Kondensator zum zumindest teilweisen Kondensieren des so erzeugten Brüdenstroms. Bevorzugt umfasst die Destillationsvorrichtung darüber hinaus mindestens eine Destillationskolonne mit oder ohne Trennwand. Dem Fachmann bekannt und bevorzugt zu verwenden sind Einbauten in der Destillationskolonne, wie beispielsweise Füllkörper, Glockenböden, Siebböden, Dual-Flow-Böden, strukturierte Packungen, etc., beispielsweise zur Verbesserung der Trennleistung. Erfindungsgemäß besonders bevorzugt werden solche Einbauten verwendet, die einen geringen Druckverlust verursachen, wie beispielsweise strukturierte Packungen, Füllkörper oder Dual-Flow-Böden.

Die erfindungsgemäß eingesetzte Destillationsvorrichtung ist mit einer Vorrichtung zur Erzeugung eines Unterdrucks in der Destillationsvorrichtung, bevorzugt fluidisch, verbunden. Diese Vorrichtung umfasst erfindungsgemäß einen Flüssigkeitsringverdichter. Flüssigkeitsringverdichter haben für die Destillation von Isocyanaten aus der Phosgenierung große Vorteile gegenüber anderen Verdichtern wie beispielsweise trockenlaufenden Verdichtern. So sind sie beispielsweise unempfindlich gegenüber in der Flüssigkeit mit gerissenen Tröpfchen und neigen nicht zu Anbackungen, beispielsweise durch Zersetzung von mit dem Brüdenstrom eingebrachten organischen Verbindungen. Gleichzeitig können sie auch als Gaswäscher fungieren, so dass z.B. im Brüdenstrom enthaltenes Phosgen zumindest teilweise ausgewaschen und ein unbedenklicherer Abgasstrom erhalten wird. Allerdings ist der durch den Flüssigkeitsringverdichter erzielbare Unterdruck (Saugdruck) durch das Funktionsprinzip begrenzt.

Sofern erfindungsgemäß in der Destillationsvorrichtung ein besonders niedriger Druck, beispielsweise 0,1 bis 30 mbar(a), eingestellt werden soll, kann deshalb zwischen Destillationsvorrichtung und dem vorhandenen mindestens einen Flüssigkeitsringverdichter mindestens ein oder mehrere Vorverdichter mit dem gleichen oder einem anderen Funktionsprinzip eingesetzt werden. Beispielsweise seien hier Gasstrahler, Schraubenverdichter, Gasringpumpen oder Drehkolbengebläse genannt. Bei einer solchen Ausführungsform kann es vorteilhaft sein, zwischen Vorverdichter und Flüssigkeitsringverdichter eine Kondensation durchzuführen, um den Förderstrom für den Flüssigkeitsringverdichter zu minimieren.

In einer weiteren Ausführungsform der Erfindung sind mehrere Destillationsvorrichtungen in analoger Weise, bevorzugt fluidisch, mit dem mindestens einen Flüssigkeitsringverdichter verbunden. Diese Destillationsvorrichtungen können beispielsweise ausgewählt sein aus der Gruppe bestehend aus einer Destillationsvorrichtung zur Abtrennung des Lösungsmittels aus dem bereits von Phosgen und Chlorwasserstoff befreiten Reaktionsaustrag, einer Destillationsvorrichtung zur Aufbereitung des Reaktionslösungsmittels, einer Destillationsvorrichtung zur Rückstandsaufkonzentrierung, einer Destillationsvorrichtung zur Abtrennung von weiteren Leichtsiedern aus dem bereits von Phosgen, Chlorwasserstoff und Lösungsmitteln befreiten Roh-Isocyanat-Konzentrat und einer Vorrichtung zur Reindestillation des Roh-Isocyanates.

In einer bevorzugten Ausführungsform werden die Abgase mehrerer Destillationsvorrichtungen in einer Sammelleitung zusammengeführt und von dort gemeinsam in den Ansaugstutzen des mindestens einen Flüssigkeitsringverdichters geleitet. Die Abgasströme aus den verschiedenen Destillationsvorrichtungen können jeweils nach der Kondensation der Brüden einer Gaswäsche unterzogen werden. Die Abgasströme können einzeln oder auch in beliebigen Kombinationen gemeinsam gewaschen werden. Bevorzugt werden einzelne Gasströme gewaschen, bevor das Abgas in der Sammelleitung vereinigt wird.

Als Gaswäscher eignen sich im Grunde alle Apparate, in denen ein Gasstrom mit einem Flüssigkeitsstrom in Kontakt gebracht werden kann, also beispielsweise Tauchwäscher, Sprühwäscher, Füllkörperkolonnen, Wirbelwäscher oder Rotationswäscher bevorzugt sind solche, die einen geringen Druckverlust für das durchströmende Gas aufweisen, beispielsweise Füllkörperkolonnen oder Sprühwäscher. Das nicht kondensierte Abgas wird vorzugsweise von unten nach oben durch den Gaswäscher geleitet, während das Lösungsmittel den Wäscher im Gegenstrom, d.h. von oben nach unten, durchläuft.

Bevorzugte Lösungsmittel, die beim Waschen des Gasstroms eingesetzt werden, sind die bereits genannten Lösungsmittel, die auch in der Herstellung des Isocyanates zum Einsatz kommen können, beispielsweise Chlorbenzol, Dichlorbenzol, insbesondere o-Dichlorbenzol, Xylol, 1-Chlor-2,4-dimethylbenzol, Chlortoluol, insbesondere o- oder m-Chlortoluol, oder deren Gemische, bevorzugt Chlorbenzol und/oder o-Dichlorbenzol. Ganz besonders bevorzugt wird für das Waschen des Gasstroms das gleiche Lösungsmittel verwendet, das auch in der Herstellung des Isocyanates eingesetzt wurde. Die Temperatur des Lösungsmittels liegt bevorzugt bei -20 und + 10 °C, besonders bevorzugt ist die Temperatur dabei kleiner oder gleich der Betriebstemperatur des mindestens einen Flüssigkeitsringverdichters. Auf diese bevorzugte Weise werden leicht flüchtige organische Verbindungen und Phosgen zumindest teilweise aus dem Abgasstrom ausgewaschen, so dass ein vorgereinigter Abgasstrom erhalten wird. Chlorwasserstoff lässt sich unter diesen Bedingungen hingegen nur sehr begrenzt aus dem Abgasstrom entfernen und verbleibt deshalb weitgehend im Abgas. Häufig stellt es sogar dessen Hauptbestandteil dar.

Es ist erfindungsgemäß auch möglich, den Gehalt an Chlorwasserstoff durch Waschen mit geeigneten anorganischen Waschmedien, beispielsweise einer Lösung von Silbersulfat in konzentrierter Schwefelsäure, abzusenken.

Erfindungsgemäß wird der mindestens eine Flüssigkeitsringverdichter im Allgemeinen mit einem Druck p (Saugdruck) von 10 bis 200 mbar(a), bevorzugt 20 bis 150 mbar(a) und besonders bevorzugt 30 bis 120 mbar(a), betrieben.

Der genannte Druckbereich ist für viele Destillationsaufgaben bei der Reinigung von Roh-Isocyanaten ausreichend und ermöglicht die Destillation unter weitgehender Vermeidung von Zersetzungsreaktionen. Sofern erfindungsgemäß ein niedrigerer Druck benötigt wird, kann dieser wie zuvor beschrieben durch den Einsatz eines Vorverdichters mit anderem oder gleichem Funktionsprinzip erreicht werden.

Als Betriebsflüssigkeit zur Erzeugung des Flüssigkeitsrings in dem mindestens einen Flüssigkeitsringverdichter eignen sich im erfindungsgemäßen Verfahren im Allgemeinen Lösungsmittel, wie sie auch in der Herstellung des Isocyanates eingesetzt werden können, d.h. beispielsweise Chlorbenzol, Dichlorbenzol, insbesondere o-Dichlorbenzol, Xylol, 1-Chlor-2,4-dimethylbenzol, Chlortoluol, insbesondere o- oder m-Chlortoluol, oder deren Gemische, bevorzugt Chlorbenzol und/oder o-Dichlorbenzol. Diese Betriebsflüssigkeiten sind einerseits unter den Betriebsbedingungen weitgehend inert und haben für den Einsatz als Betriebsflüssigkeit vorteilhafte Schmelz- und Siedepunkte. Besonders bevorzugt wird als Betriebsflüssigkeit das gleiche Lösungsmittel verwendet, das auch in der Herstellung des Isocyanates eingesetzt wird. Auf diese Weise kann eine zusätzliche Aufbereitung des Betriebsmittels entfallen, da diese zusammen mit dem in der Herstellung verwendeten Lösungsmittel erfolgen kann.

Unter der Betriebstemperatur des mindestens einen Flüssigkeitsringverdichters ist vorliegend die Temperatur der am Druckstutzen austretenden Flüssigkeit zu verstehen. Die Betriebstemperatur des mindestens einen Flüssigkeitsringverdichters liegt im erfindungsgemäßen Verfahren im Allgemeinen bei -17 bis +15 °C. Höhere Temperaturen erhöhen den Dampfdruck der Betriebsflüssigkeit und senken so den möglichen Saugdruck beim Betrieb des mindestens einen Flüssigkeitsringverdichters. Bei niedrigeren Betriebstemperaturen steigen der energetische und apparative Aufwand für die Kühlung der Betriebsflüssigkeit und deren Viskosität. Letzteres führt zu einem weiteren Anstieg des ohnehin schon hohen Energieverbrauchs für die Förderung der Betriebsflüssigkeit. Bevorzugt liegt die Betriebstemperatur des Flüssigkeitsringverdichters bei -15 bis +12 °C, besonders bevorzugt bei -12 bis +10 °C.

Auch wenn das Reaktionsprodukt aus der Phosgenierung zuvor bereits von Chlorwasserstoff und Phosgen befreit wurde, enthalten die bei der destillativen Reinigung des Roh-Isocyanates gemäß dem erfindungsgemäßen Verfahren anfallenden Abgase der Destillationsvorrichtung üblicherweise noch geringe Mengen an Phosgen. Dies kann einerseits an einer nicht vollständigen Abtrennung des Phosgens in den vorherigen Destillationsschritten liegen, es kann aber beispielsweise auch durch die Abspaltung von Phosgen aus chlorhaltigen, schwersiedenden Nebenkomponenten entstehen. Dieses Phosgen kann durch die erfindungsgemäße Verwendung mindestens eines Flüssigkeitsringverdichters zur Erzeugung eines Vakuums in der Destillationsvorrichtung in vorteilhafter Weise zumindest teilweise ausgewaschen werden. Auf diese Weise wird die Menge an Phosgen im Abgas reduziert, so dass es sicherer gehandhabt und einfacher entsorgt werden kann.

Das Phosgen wird dabei bevorzugt zumindest teilweise in der Betriebsflüssigkeit gelöst, so dass diese am Austritt aus dem mindestens einen Flüssigkeitsringverdichter vorzugsweise einen Gehalt an Phosgen von 0,001 bis 4,5 Gew.-%, bevorzugt 0,005 bis 2 Gew.-%, besonders bevorzugt 0,01 bis 1 Gew.-% und am meisten bevorzugt 0,05 bis 0,5 Gew.-% aufweist. Geringere Gehalte an Phosgen erfordern einen unverhältnismäßig hohen Einsatz von frischer, phosgenarmer oder phosgenfreier Betriebsflüssigkeit, welche anschließend aufbereitet werden muss. Eine Anreicherung auf höhere Gehalte führt zu einer unerwünschten Erhöhung der Gesamtmenge des in der Anlage befindlichen Gefahrstoffs Phosgen und erhöht das Risiko für das Auftreten von Kavitation an dem mindestens einen Flüssigkeitsringverdichter.

Es wurde nun allerdings erfindungsgemäß gefunden, dass es auch bei Einhaltung niedriger Phosgen-Gehalte in der Betriebsflüssigkeit häufig zu Problemen mit Kavitation kommt, wenn der Gehalt an aciden Chlorverbindungen, auch als AC-Wert, AC-Gehalt oder einfach Acidität bezeichnet, ansteigt. Die Bestimmung des AC-Wertes erfolgt gemäß ASTM D 5629 für AC-Wert < 100 ppm und gemäß ASTM D 6099 für AC-Werte > 100 ppm, wobei die Betriebsflüssigkeit das Isocyanat als Matrix ersetzt.

Für einen zuverlässigen Betrieb des mindestens einen Flüssigkeitsringverdichters ist es erfindungsgemäß erforderlich, dass die Betriebsflüssigkeit am Austritt aus dem mindestens einen Flüssigkeitsringverdichter einen AC-Wert von weniger als 35000 ppm, bevorzugt weniger als 25000 ppm und besonders bevorzugt weniger als 15000 ppm aufweist.

Die Anwesenheit von Chlorwasserstoff erhöht in besonderem Maße das Risiko für das Auftreten von Kavitation. Deshalb wird in einer bevorzugten Ausführungsform der Erfindung das Verfahren so betrieben, dass die am Druckstutzen aus dem mindestens einen Flüssigkeitsringverdichter austretende Betriebsflüssigkeit einen Gehalt von HCl aufweist, der ≤ 1,6 Gew.-% ist, bevorzugt ≤ 1 Gew.-%, besonders bevorzugt ≤ 0,5 Gew.-% und ganz besonders bevorzugt ≤ 0,2 Gew.-% ist. Der Gehalt an HCl beträgt dabei bevorzugt mindestens 1 ppm, besonders bevorzugt mindestens 10 ppm, ganz besonders bevorzugt mindestens 20 ppm und am meisten bevorzugt mindestens 50 ppm.

Auf diese Weise kann Kavitation im erfindungsgemäßen Verfahren deutlich abgemildert oder gar gänzlich vermieden werden. Dem Fachmann ist dabei bekannt, dass für das Auftreten von Kavitation gegebenenfalls die Betriebstemperatur, der Saugdruck sowie ein eventueller Gasballast mit einem Inertgas ebenfalls eine Rolle spielen. Eine höhere Betriebstemperatur, ein geringerer Saugdruck und ein geringer oder fehlender Gasballast wirken sich negativ aus, so dass es sich für solche Fälle empfiehlt, den HCl-Gehalt stärker einzuschränken. Darüber hinaus spielt, wie bereits erwähnt, auch der Phosgen-Gehalt in der Betriebsflüssigkeit eine Rolle. Mit steigendem Phosgen-Gehalt in der Betriebsflüssigkeit nimmt die Kavitationsneigung zu, so dass es wiederum vorteilhaft ist, einen umso geringeren Chlorwasserstoffgehalt einzustellen.

In einer bevorzugten Ausführungsform betrifft die vorliegende Erfindung das erfindungsgemäße Verfahren, wobei in der Betriebsflüssigkeit am Austritt aus dem mindestens einen Flüssigkeitsringverdichter der Gehalt an Phosgen $c(COCl_2)$ 0,001 bis 4,5 Gew.-%, bevorzugt 0,005 bis 2 Gew.-%, besonders bevorzugt 0,01 bis 1 Gew.-%, am meisten bevorzugt 0,05 bis 0,5 Gew.-%, beträgt, und der Gehalt an Chlorwasserstoff $c(HCl) \leq 1,6$ Gew.-%, bevorzugt $\leq 1$ Gew.-%, besonders bevorzugt $\leq 0,5$ Gew.-% und ganz besonders bevorzugt $\leq 0,2$ Gew.-%, ist.

In einer besonders bevorzugten Ausführungsform der Erfindung, liegt der Gehalt an Chlorwasserstoff in der Betriebsflüssigkeit beim Austritt aus dem Verdichter deshalb unterhalb eines Maximalgehalts $c_{max}(HCl)$ gemäß der allgemeinen Formel (I), wobei für Formelergebnisse unterhalb von 0,01% ein Maximalgehalt $c_{max}(HCl)$ von 0,01 Gew.-% gilt:

$$c_{max}(HCl) = p_{saug} / 10\text{^}(6,115 - 900/(T+279)) - c(COCl_2)/100 \qquad (I),$$

wobei $c_{max}(HCl)$, $p_{saug}$, T und $c(COCl_2)$ die folgenden Bedeutungen haben

| | |
|---|---|
| $c_{max}(HCl)$ | maximaler Gehalt von Chlorwasserstoff in der Betriebsflüssigkeit in Gew.-% |
| $p_{saug}$ | Ansaugdruck des Flüssigkeitsringverdichters in mbar(a) |
| T | Betriebstemperatur des Flüssigkeitsringverdichters in °C |
| $c(COCl_2)$ | Gehalt von Phosgen in der Betriebsflüssigkeit in Gew.-% |

Die Gehalte von Phosgen und HCl in der Betriebsflüssigkeit können erfindungsgemäß auf verschiedenen Wegen und teilweise unabhängig voneinander beeinflusst werden. Wie zuvor schon beschrieben, ist es beispielsweise möglich, zumindest einen Abgasstrom aus einer Destillationsvorrichtung, die mit dem Flüssigkeitsringverdichter, bevorzugt fluidisch, verbunden ist, der also zur Erzeugung des Vakuums in dieser Destillationsvorrichtung beiträgt, einer Gaswäsche in einem Gaswäscher zu unterziehen. Je nach verwendeter Waschflüssigkeit und Betriebsbedingungen wie Temperatur, Sättigung, Flüssigkeitsmenge usw., kann damit der Eintrag an Phosgen und/oder Chlorwasserstoff in den Flüssigkeitsringverdichter beeinflusst werden. Alternativ kann die Betriebsflüssigkeit selbst behandelt werden, indem sie zum Beispiel mit einem Inertgas gestrippt wird. Dies kann sowohl in dem mindestens einen Flüssigkeitsringverdichter selbst als auch extern in einer separaten Vorrichtung erfolgen.

In einer weiteren, bevorzugten Ausführungsform des erfindungsgemäßen Verfahrens werden die Gehalte an Chlorwasserstoff und/oder Phosgen in der Betriebsflüssigkeit beeinflusst, indem gebrauchte Betriebsflüssigkeit zumindest teilweise gegen frische bzw. aufbereitete Betriebsflüssigkeit ersetzt wird. Einen Sonderfall dieser Ausführungsform stellt ein Verfahren dar, bei dem die Betriebsflüssigkeit nach dem Austritt aus dem Flüssigkeitsringverdichter aufgeheizt wird, wobei zuvor gelöste leichtflüchtige Verbindungen ausgetrieben und abgetrennt werden, und dann wieder zurückgekühlt wird, um erneut als Betriebsflüssigkeit in den Flüssigkeitsringverdichter eingebracht zu werden.

Mit dem komprimierten Gas wird am Druckstutzen auch ein Teil der Betriebsflüssigkeit ausgestoßen. In einer bevorzugten Ausführungsform des erfindungsgemäßen Verfahrens wird die am Druckstutzen austretende Flüssigkeit, bevorzugt enthaltend Gas- und Betriebsflüssigkeit, nach Austritt aus dem mindestens einen Flüssigkeitsringverdichter in einem Flüssigkeitsabscheider getrennt. Bevorzugt wird dann zumindest ein Teil der abgeschiedenen Betriebsflüssigkeit, vorzugsweise nach Kühlung in einem Kühler, über eine Rückführungsleitung rezirkuliert, d.h. zum Eintrittsstutzen für Betriebsflüssigkeit zurückgeführt. Über die erfindungsgemäß bevorzugt erfolgende Kühlung lässt sich die beispielsweise durch Verdichtung, Kondensation und Absorption entstehende Wärme vorteilhaft abführen und die Betriebstemperatur im gewünschten Bereich einstellen.

Vorzugsweise wird ein Teilstrom der Betriebsflüssigkeit entweder kontinuierlich oder diskontinuierlich aus dem System entfernt und durch frische oder aufbereitete Betriebsflüssigkeit, im Folgenden als Ersatz-Betriebsflüssigkeit bezeichnet, ersetzt. Auf diese Weise lässt sich der Phosgen-Gehalt in der Betriebsflüssigkeit am Austritt aus dem mindestens einen Flüssigkeitsringverdichter vorteilhaft auf die erfindungsgemäßen Bereiche einstellen. Die Ersatz-Betriebsflüssigkeit kann an verschiedenen Stellen in das System eingebracht werden. Sie kann beispielsweise in die Rückführungsleitung eingebracht werden, über einen separaten Betriebsflüssigkeitsstutzen an dem mindestens einen Flüssigkeitsringverdichter selbst zugeführt werden oder in einen Flüssigkeitsabscheider eingebracht werden. Bevorzugt wird die Ersatz-Betriebsflüssigkeit vor dem Einbringen in die Betriebsflüssigkeit auf eine Temperatur gebracht, die maximal 20 °C, bevorzugt maximal 15 °C, besonders bevorzugt maximal 10 °C und ganz besonders bevorzugt maximal 5 °C, oberhalb der Betriebstemperatur des mindestens einen Flüssigkeitsringverdichters liegt. Die Ersatz-Betriebsflüssigkeit weist vorzugsweise einen Gehalt an Phosgen von $\leq 1000$ ppm, bevorzugt $\leq 100$ ppm, besonders bevorzugt $\leq 20$ ppm auf. Außerdem enthält die Ersatz-Betriebsflüssigkeit vorzugsweise $\leq 250$ ppm, bevorzugt $\leq 100$ ppm, besonders bevorzugt $\leq 50$ ppm an Chlorwasserstoff. Chlorwasserstoff-Gehalte < 1 ppm, vorzugsweise < 5 ppm, besonders bevorzugt < 10 ppm tragen nicht mehr wesentlich zu einer Verbesserung des Verfahrens bei und sind deshalb nicht erforderlich.

Die vorliegende Erfindung betrifft daher bevorzugt das erfindungsgemäße Verfahren, wobei zumindest ein Teil der Betriebsflüssigkeit dem mindestens einen Flüssigkeitsrindverdichter kontinuierlich entnommen, auf eine Temperatur, die maximal 20 °C, bevorzugt maximal 15 °C, besonders bevorzugt maximal 10 °C und ganz besonders bevorzugt maximal 5 °C, oberhalb der Betriebstemperatur des mindestens einen Flüssigkeitsringverdichters liegt, abgekühlt und anschließend dem mindestens einen Flüssigkeitsringverdichter kontinuierlich wieder zugeführt wird.

In einer bevorzugten Ausführungsform enthält die Ersatz-Betriebsflüssigkeit ein Lösungsmittel, bevorzugt besteht sie aus einem Lösungsmittel, das bereits in einer Phosgenierung eingesetzt und vorzugsweise durch Destillation aufbereitet wurde. In diesem Fall ist dessen Phosgen-Gehalt vorzugsweise ≤ 1000 ppm, bevorzugt ≤ 100 ppm und besonders bevorzugt ≤ 20 ppm, jedoch gleichzeitig bevorzugt ≥ 1 ppm, besonders bevorzugt ≥ 5 ppm und besonders bevorzugt ≥ 10 ppm. Eine weitere Senkung des Phosgen-Gehalts würde einen unverhältnismäßig hohen Aufwand zur Reinigung des Lösungsmittels erfordern.

Die vorliegenden Erfindung betrifft daher bevorzugt das erfindungsgemäße Verfahren, wobei die Betriebsflüssigkeit in dem mindestens einen Flüssigkeitsringverdichter zumindest teilweise, bevorzugt kontinuierlich, durch Betriebsflüssigkeit mit einem Phosgen-Gehalt von ≤ 1000 ppm, bevorzugt ≤ 100 ppm, besonders bevorzugt ≤ 20 ppm, ausgetauscht wird.

Die vorliegende Erfindung betrifft auch das Isocyanat, erhältlich, bevorzugt erhalten, durch das erfindungsgemäße Verfahren.

**Beispiele:**

Methode zur Bestimmung des AC-Wertes (Acidität) der Betriebsflüssigkeit:

Die Bestimmung des AC-Wertes (Acidität) erfolgt gemäß ASTM D 5629 für AC-Werte < 100 ppm und gemäß ASTM D 6099 für AC-Werte > 100 ppm, wobei die jeweilige Betriebsflüssigkeit das in der Methode genannte Isocyanat als Matrix ersetzt.

Methode zur Bestimmung des Phosgen-Gehalts in der Betriebsflüssigkeit:

Der Phosgen-Gehalt der Betriebsflüssigkeit kann mittels Infrarot-Spektroskopie bestimmt werden. Dazu werden Extinktionsspektren des entsprechenden reinen Betriebsmittels sowie der Probe der Betriebsflüssigkeit gemessen. Anschließend wird die Intensität der Carbonyl-Bande des Phosgens bei etwa 1806 $cm^{-1}$ in dem Spektrum der Betriebsflüssigkeit nach Subtraktion des Spektrums des reinen Betriebsmittels gemessen. Die Ermittlung des Phosgen-Gehalts erfolgt mittels einer Kalibrierung mit Proben mit jeweils bekanntem Phosgen-Gehalt unter Berücksichtigung des Ursprungs. Die Messung erfolgt in $CaF_2$-Küvetten geeigneter Schichtdicke, so dass die Extinktion bei Bedarf nach Verdünnung im Bereich von 0,01 bis 1,50 liegt, mit einer Auflösung von 4 $cm^{-1}$ und mindestens 4 Scans.

Bestimmung des HCl-Gehalts in der Betriebsflüssigkeit:

Der HCl-Gehalt (Chlorwasserstoff-Gehalt) der Betriebsflüssigkeit ergibt sich vorliegend als Differenz aus dem Phosgen-Gehalt und dem AC-Wert (Acidität) der Betriebsflüssigkeit.

Beispiel 1:

Hexamethylendiisocyanat, welches aus einer Gasphasen-Phosgenierung von Hexamethylendiamin erhalten wurde und aus dem bereits Chlorwasserstoff, Phosgen, Chlorbenzol und leichtsiedende Nebenkomponenten weitgehend in einem mehrstufigen Destillationsverfahren entfernt wurden, wurde als Roh-Isocyanat einer kontinuierlichen Reindestillation im Vakuum unterzogen. Das Roh-Isocyanat hatte einen HC-Gehalt gemäß ISO 15028:2014 von ca. 8000 ppm. Die Destillation erfolgte in einer Kolonne mit internem Kondensator bei einem Kopfdruck von 50 mbar(a). Die am Kopf der Kolonne austretenden Brüden wurden in einem Gaswäscher mit Chlorbenzol gewaschen. Der gewaschene Brüdenstrom wurde in einem Kühler auf -11 °C abgekühlt und zur Erzeugung des Unterdrucks mit einem Flüssigkeitsringverdichter angesaugt. Ein direkt vor dem Ansaugstutzen installierter Drucksensor zeigte einen Saugdruck von 30 mbar an. Der Flüssigkeitsringverdichter wurde mit Chlorbenzol als Betriebsflüssigkeit bei einer Betriebstemperatur von -2 °C im Umlaufflüssigkeitsbetrieb betrieben, d.h. die austretende Betriebsflüssigkeit wurde im Kreislauf über einen Kühler wieder zum Eintrittsstutzen für Betriebsflüssigkeit zurückgeführt. Dabei wurde kontinuierlich ein Teil der Betriebsflüssigkeit ausgeschleust und durch redestilliertes Chlorbenzol mit einem Phosgen-Gehalt von 10 ppm und einem Chlorwasserstoff-Gehalt von 2 ppm ersetzt. Die auszutauschende Menge an Betriebsflüssigkeit wurde in regelmäßigen Abständen so angepasst, dass sich in der druckseitig austretenden Betriebsflüssigkeit ein AC-Wert von 2150 ppm einstellte. Der Phosgen-Gehalt wurde zu 0,25 Gew.-% bestimmt und entsprechend betrug der errechnete Chlorwasserstoff-Gehalt 0,03 Gew.-%.

Der Flüssigkeitsringverdichter konnte ohne Kavitationsprobleme dauerhaft betrieben werden.

Beispiel 2:

Die Destillation wurde analog zu Beispiel 1 betrieben, jedoch wurde diesmal der Abgasstrom mit dem Abgasstrom aus einer Kolonne zur Lösemittelabtrennung aus dem Reaktionsprodukt in einer Sammelleitung vereinigt. Der Abgasstrom aus der Lösungsmittelabtrennung hatte Chlorwasserstoff als Hauptbestandteil und enthielt daneben noch Chlorbenzol und Spuren von Phosgen. Mit dem vereinigten Abgasstrom aus der Sammelleitung wurde dann weiter verfahren wie mit dem gewaschenen Brüdenstrom aus Beispiel 1.

Die Austauschrate der Betriebsflüssigkeit des Flüssigkeitsringverdichters wurde so gewählt, dass sich in der druckseitig austretenden Betriebsflüssigkeit ein AC-Wert von 9000 ppm einstellte. Der Phosgen-Gehalt wurde zu 0,007 Gew.-% bestimmt, und entsprechend betrug der errechnete Chlorwasserstoffgehalt 0,9 Gew.-%.

Der Flüssigkeitsringerdichter konnte dauerhaft betrieben werden, wobei jedoch zeitweise leichte Kavitationsgeräusche vernehmbar waren.

Beispiel 3:

Ausgehend von dem Betriebszustand in Beispiel 2 wurde in einem Betriebsversuch die Austauschrate der Betriebsflüssigkeit gesenkt, wobei der AC-Wert der druckseitig austretenden Betriebsflüssigkeit auf 18100 ppm anstieg. Der Phosgen-Gehalt wurde zu 0,014 Gew.-% bestimmt, und entsprechend betrug der errechnete Chlorwasserstoffgehalt 1,8 Gew.-%.

Beim Betrieb des Flüssigkeitsringverdichters auf diese Art kam es zu stärkeren Kavitationsgeräuschen, Vibrationen und einem Anstieg des am Saugstutzen gemessenen Drucks.

Durch eine Absenkung der Betriebstemperatur auf -12 °C und eine Erhöhung des Drucks im Ansaugstutzen auf 80 mbar(a) konnten die Probleme beseitigt werden.

Beispiel 4:

Toluylendiisocyanat, welches aus einer Gasphasen-Phosgenierung von Toluylendiamin erhalten wurde und aus dem bereits Chlorwasserstoff, Phosgen und o-Dichlorbenzol in einem mehrstufigen Destillationsverfahren weitgehend entfernt wurden, wurde als Roh-Isocyanat einer kontinuierlichen Reindestillation im Vakuum unterzogen. Das Roh-Isocyanat hatte einen HC-Gehalt gemäß ISO 15028:2014 von ca. 12000 ppm. Die Destillation erfolgte in einer Trennwandkolonne mit internem Kondensator bei einem Kopfdruck von 135 mbar(a), wobei am Kopf der Kolonne ein Strom enthaltend leichtsiedende Nebenkomponenten und o-Dichlorbenzol entnommen wurde und das gereinigte Toluylendiisocyanat als Seitenstrom anfiel. Die am Kopf der Kolonne austretenden Brüden wurden in einem Gaswäscher mit o-Dichlorbenzol gewaschen. Der gewaschene Brüdenstrom wurde in einem Kühler auf 2 °C abgekühlt und zur Erzeugung des Unterdrucks mit einem Flüssigkeitsringverdichter angesaugt. Ein direkt vor dem Ansaugstutzen installierter Drucksensor zeigte einen Saugdruck von 120 mbar an. Der Flüssigkeitsringverdichter wurde mit o-Dichlorbenzol als Betriebsflüssigkeit bei einer Betriebstemperatur von 7 °C im Umlaufflüssigkeitsbetrieb betrieben, d.h. die austretende Betriebsflüssigkeit wurde im Kreislauf über einen Kühler wieder zum Eintrittsstutzen für Betriebsflüssigkeit zurückgeführt. Dabei wurde kontinuierlich ein Teil der Betriebsflüssigkeit ausgeschleust und durch redestilliertes o-Dichlorbenzol mit einem Phosgen-Gehalt von 10 ppm und einem Chlorwasserstoff-Gehalt von 2 ppm ersetzt. Die auszutauschende Menge an Betriebsflüssigkeit wurde in regelmäßigen Abständen so angepasst, dass sich in der druckseitig austretenden Betriebsflüssigkeit ein AC-Wert von 950 ppm einstellte. Der Phosgen-Gehalt wurde zu 0,02 Gew.-% bestimmt, und entsprechend betrug der errechnete Chlorwasserstoff-Gehalt 0,08 Gew.-%.

Der Flüssigkeitsringverdichter konnte ohne Kavitationsprobleme dauerhaft betrieben werden.

Vergleichsbeispiel 5:

Pentamethylendiisocyanat, welches aus einer Phosgenierung von Pentamethylendiamin erhalten wurde und aus dem bereits Chlorwasserstoff, Phosgen, Chlorbenzol und leichtsiedende Nebenkomponenten weitgehend in einem mehrstufigen Destillationsverfahren entfernt wurden, wurde als Roh-Isocyanat einer kontinuierlichen Reindestillation im Vakuum unterzogen. Das Roh-Isocyanat hatte einen HC-Gehalt gemäß ISO 15028:2014 von ca. 13000 ppm. Die Destillation erfolgte in einer Kolonne mit internem Kondensator bei einem Kopfdruck von 45 mbar(a). Die am Kopf der Kolonne austretenden Brüden wurden ohne vorherigen Einsatz von Gaswäschern in einem Kühler auf -12 °C abgekühlt und zur Erzeugung des Unterdrucks mit einem Flüssigkeitsringverdichter angesaugt. Ein direkt vor dem Ansaugstutzen installierter Drucksensor zeigte einen Saugdruck von 30 mbar an. Der Flüssigkeitsringverdichter wurde mit Chlorbenzol als Betriebsflüssigkeit bei einer Betriebstemperatur von -9 °C im Umlaufflüssigkeitsbetrieb betrieben, d.h. die austretende Betriebsflüssigkeit wurde im Kreislauf über einen Kühler wieder zum Eintrittsstutzen für Betriebsflüssigkeit zurückgeführt. Dabei wurde kontinuierlich ein Teil der Betriebsflüssigkeit ausgeschleust und durch redestilliertes Chlorbenzol mit einem Phosgen-Gehalt von 10 ppm und einem Chlorwasserstoff-Gehalt von 2 ppm ersetzt.

Es kam zu starken Kavitationsgeräuschen, Vibrationen und einem Anstieg des Saugdrucks. Eine Analyse der druckseitig austretenden Betriebsflüssigkeit ergab einen AC-Wert von 38600 ppm. Der Phosgen-Gehalt wurde zu 2,8 Gew.-% bestimmt und entsprechend betrug der errechnete Chlorwasserstoff-Gehalt 1,8 Gew.-%. Auch durch eine Absenkung der Betriebstemperatur auf -14 °C konnten die Probleme nicht behoben werden.

Daraufhin wurde die Austauschrate der Betriebsflüssigkeit verdreifacht, so dass der AC-Wert auf ca. 12900 ppm sank, und der Betrieb normalisierte sich, so dass auch die Betriebstemperatur ohne weitere Probleme wieder auf -9 °C angehoben werden konnte.

**Patentansprüche**

1. Verfahren zur destillativen Aufreinigung eines durch Phosgenierung des entsprechenden Amins erhaltenen Roh-Isocyanates in einer Destillationsapparatur im Vakuum, um das entsprechende Isocyanat zu erhalten, **dadurch gekennzeichnet, dass**

   - mindestens ein Flüssigkeitsringverdichter eingesetzt wird, um das Vakuum in der Destillationsapparatur zu erzeugen,
   - eine Betriebsflüssigkeit ausgewählt aus der Gruppe bestehend aus Chlorbenzol, Dichlorbenzol, Xylol, 1-Chlor-2,4-dimethylbenzol, Chlortoluol und Mischungen davon für den mindestens einen Flüssigkeitsringverdichter verwendet wird,
   - der Druck p auf der Ansaugseite des mindestens einen Flüssigkeitsringverdichters 10 bis 200 mbar(a) beträgt,
   - die Betriebstemperatur des mindestens einen Flüssigkeitsringverdichters -17 bis +15 °C beträgt, und
   - die Betriebsflüssigkeit am Austritt aus dem mindestens einen Flüssigkeitsringverdichter einen AC-Wert von weniger als 35000 ppm aufweist.

2. Verfahren nach Anspruch 1, **dadurch gekennzeichnet, dass** in der Betriebsflüssigkeit am Austritt aus dem mindestens einen Flüssigkeitsringverdichter der Gehalt an Phosgen $c(COCl_2)$ 0,001 bis 4,5 Gew.-%, bevorzugt 0,005 bis 2 Gew.-%, besonders bevorzugt 0,01 bis 1 Gew.-%, am meisten bevorzugt 0,05 bis 0,5 Gew.-%, beträgt, und der Gehalt an Chlorwasserstoff $c(HCl) \leq 1,6$ Gew.-%, bevorzugt $\leq 1$ Gew.-%, besonders bevorzugt $\leq 0,5$ Gew.-% und ganz besonders bevorzugt $\leq 0,2$ Gew.-%, ist.

3. Verfahren nach Anspruch 1 oder 2, **dadurch gekennzeichnet, dass** zumindest ein Teil der Betriebsflüssigkeit dem mindestens einen Flüssigkeitsrindverdichter kontinuierlich entnommen, auf eine Temperatur, die maximal 20 °C, bevorzugt maximal 15 °C, besonders bevorzugt maximal 10 °C und ganz besonders bevorzugt maximal 5 °C, oberhalb der Betriebstemperatur des mindestens einen Flüssigkeitsringverdichters liegt, abgekühlt und anschließend dem mindestens einen Flüssigkeitsringverdichter kontinuierlich wieder zugeführt wird.

4. Verfahren nach einem der Ansprüche 1 bis 3, **dadurch gekennzeichnet, dass** das Roh-Isocyanat aus einer Gasphasen-Phosgenierung oder einer Flüssigphasen-Phosgenierung stammt.

5. Verfahren nach einem der Ansprüche 1 bis 4, **dadurch gekennzeichnet, dass** Betriebsflüssigkeit in dem mindestens einen Flüssigkeitsringverdichter zumindest teilweise, bevorzugt kontinuierlich, durch Betriebsflüssigkeit mit einem Phosgen-Gehalt von $\leq 1000$ ppm, bevorzugt $\leq 100$ ppm, besonders bevorzugt $\leq 20$ ppm, ausgetauscht wird.

6. Verfahren nach einem der Ansprüche 1 bis 5, **dadurch gekennzeichnet, dass** das Isocyanat ausgewählt ist aus der Gruppe bestehend aus Monoisocyanaten, insbesondere R,S-1-Phenylethylisocyanat, 1-Methyl-3-Phenylpropylisocyanat, Pentylisocyanat, 6-Methyl-2-heptanisocyanat, Cyclopentylisocyanat oder 3-(Methylthio)phenylisocyanat, Diisocyanaten, insbesondere Hexamethylendiisocyanat (HDI), Pentamethylendiisocyanat (PDI), 1,4-Butandiisocyanat, 1,8-Diisocyanatooctan, 1,9-Diisocyanatononan, 2-Methylpentamethylendiisocyanat, 2,2-Dimethylpentamethylendiisocyanat, Neopentandiisocyanat, 2,2,4-Trimethylhexamethylendiisocyanat und 2,4,4-Trimethylhexamethylendiisocyanat, 3-Isocyanatmethyl-3,5,5-trimethylcyclohexylisocyanat (IPDI), 2,4' und 4,4'-Diisocyanatodicyclohexylmethan ($H_{12}$MDI), 4,4'-Diisocyanato-3,3'-dimethyldicyclohexylmethan, 2,4- und 2,6-Diisocyanato-methylcyclohexan ($H_6$TDI), 1,3-Cyclohexandiisocyanat, 1,4-Cyclohexandiisocyanat, 1,3-Diisocyanato-2-methylcyclohexan, 1,3-Diisocyanato-4-methyl-cyclohexan, 1,4-Diisocyanato-3,3,5-trimethylcyclohexan, 1,3-Bis(isocyanatomethyl)-cyclohexan ($H_6$XDI), 1,4-Bis(isocyanatomethyl)cyclohexan, die Isomere des Bis(isocyanatomethyl)bicyclo[2.2.1]heptans (NBDI), Xylylendiisocyanat (XDI), insbesondere 1,3-Xylylendiisocyanat (m-XDI) oder 1,4-Xylylendiisocyanat (p-XDI), 1,3-Bis(1-isocyanato-1-methylethyl)benzol (m-TMXDI), 1,4-Bis(1-isocyanato-1-methylethyl)benzol (p-TMXDI), monomeres Diphenylmethandiisocyanat, 2,4-Toluylendiisocyanat, 2,6- Toluylendiisocyanat, 1,5-Naphthalindiisocyanat oder 1,4-Phenylendiisocyanat, Triisocyanaten, insbesondere 4-Isocyanatomethyl-1,8-octandiisocyanat, und Mischungen davon.

7. Verfahren nach einem der Ansprüche bis 6, **dadurch gekennzeichnet, dass** ein Druck p von 10 bis 200 mbar(a), bevorzugt 20 bis 150 mbar(a), besonders bevorzugt von 30 bis 120 mbar(a) erzeugt wird.

8. Isocyanat, erhältlich, bevorzugt erhalten, durch das Verfahren gemäß einem der Ansprüche bis 7.

9. Verwendung mindestens eines Flüssigkeitsringverdichters zur Erzeugung eines Vakuums in einer Vorrichtung zur Destillation eines Roh-Isocyanates, das durch Phosgenierung des entsprechenden Amins hergestellt wurde, **dadurch gekennzeichnet, dass**

    - eine Betriebsflüssigkeit ausgewählt aus der Gruppe bestehend aus Chlorbenzol, Dichlorbenzol, Xylol, 1-Chlor-2,4-dimethylbenzol, Chlortoluol und Mischungen davon für den mindestens einen Flüssigkeitsringverdichter verwendet wird,
    - der Druck p auf der Ansaugseite des mindestens einen Flüssigkeitsringverdichters 10 bis 200 mbar(a) beträgt,
    - die Betriebstemperatur des mindestens einen Flüssigkeitsringverdichters -17 bis +15 °C beträgt, und
    - die Betriebsflüssigkeit am Austritt aus dem mindestens einen Flüssigkeitsringverdichter einen AC-Wert von weniger als 35000 ppm aufweist.

10. Verwendung nach Anspruch 9, **dadurch gekennzeichnet, dass** in der Betriebsflüssigkeit am Austritt aus dem Flüssigkeitsringverdichter der Gehalt an Phosgen $c(COCl_2)$ 0,001 bis 4,5 Gew.-% beträgt, und der Gehalt an Chlorwasserstoff c(HCl) von weniger als 1,6 Gew.-% beträgt.

11. Verwendung nach Anspruch 9 oder 10, **dadurch gekennzeichnet, dass** ein Druck p von 10 bis 200 mbar(a), bevorzugt 20 bis 150 mbar(a), besonders bevorzugt von 30 bis 120 mbar(a) erzeugt wird.

12. Verfahren zum Betreiben eines Flüssigkeitsringverdichters zum Bereitstellen des Vakuums für die destillative Aufreinigung eines durch Phosgenierung des entsprechenden Amins erhaltenen Isocyanates in einer Destillationsapparatur, um das entsprechende Isocyanat zu erhalten, **dadurch gekennzeichnet, dass**

    - eine Betriebsflüssigkeit ausgewählt aus der Gruppe bestehend aus Chlorbenzol, Dichlorbenzol, Xylol, 1-Chlor-2,4-dimethylbenzol, Chlortoluol und Mischungen davon für den mindestens einen Flüssigkeitsringverdichter verwendet wird,
    - der Druck p auf der Ansaugseite des mindestens einen Flüssigkeitsringverdichters 10 bis 200 mbar(a) beträgt,
    - die Betriebstemperatur des mindestens einen Flüssigkeitsringverdichters -17 bis +15 °C beträgt, und
    - die Betriebsflüssigkeit am Austritt aus dem mindestens einen Flüssigkeitsringverdichter einen AC-Wert von weniger als 35000 ppm aufweist.

13. Verfahren nach Anspruch 12, **dadurch gekennzeichnet, dass** in der Betriebsflüssigkeit am Austritt aus dem Flüssigkeitsringverdichter der Gehalt an Phosgen $c(COCl_2)$ 0,001 bis 4,5 Gew.-% beträgt, und der Gehalt an Chlorwasserstoff c(HCl) von weniger als 1,6 Gew.-% beträgt.

14. Verfahren nach Anspruch 12 oder 13, **dadurch gekennzeichnet, dass** das Isocyanat ausgewählt ist aus der Gruppe bestehend aus Monoisocyanaten, insbesondere R,S-1-Phenylethylisocyanat, 1-Methyl-3-Phenylpropylisocyanat, Pentylisocyanat, 6-Methyl-2-heptanisocyanat, Cyclopentylisocyanat oder 3-(Methylthio)phenylisocyanat, Diisocyanaten, insbesondere Hexamethylendiisocyanat (HDI), Pentamethylendiisocyanat (PDI), 1,4-Butandiisocyanat, 1,8-Diisocyanatooctan, 1,9-Diisocyanatononan, 2-Methylpentamethylendiisocyanat, 2,2-Dimethylpentamethylendiisocyanat, Neopentandiisocyanat, 2,2,4-Trimethylhexamethylendiisocyanat und 2,4,4-Trimethylhexamethylendiisocyanat, 3-Isocyanatmethyl-3,5,5-trimethylcyclohexylisocyanat (IPDI), 2,4' und 4,4'-Diisocyanatodicyclohexylmethan ($H_{12}$MDI), 4,4'-Diisocyanato-3,3'-dimethyldicyclohexylmethan, 2,4- und 2,6-Diisocyanato-methyl-cyclohexan ($H_6$TDI), 1,3-Cyclohexandiisocyanat, 1,4-Cyclohexandiisocyanat, 1,3-Diisocyanato-2-methylcyclohexan, 1,3-Diisocyanato-4-methyl-cyclohexan, 1,4-Diisocyanato-3,3,5-trimethylcyclohexan, 1,3-Bis(isocyanatomethyl)-cyclohexan ($H_6$XDI), 1,4-Bis(isocyanatomethyl)cyclohexan, die Isomere des Bis(isocyanatomethyl)bicyclo[2.2.1]heptans (NBDI), Xylylendiisocyanat (XDI), insbesondere 1,3-Xylylendiisocyanat (m-XDI) oder 1,4-Xylylendiisocyanat (p-XDI),

13

1,3-Bis(1-isocyanato-1-methylethyl)benzol (m-TMXDI), 1,4-Bis(1-isocyanato-1-methylethyl)benzol (p-TMXDI), monomeres Diphenylmethandiisocyanat, 2,4-Toluylendiisocyanat, 2,6- Toluylendiisocyanat, 1,5-Naphthalindiisocyanat oder 1,4-Phenylendiisocyanat, Triisocyanaten, insbesondere 4-Isocyanatomethyl-1,8-octandiisocyanat, und Mischungen davon.

**Europäisches Patentamt**

**European Patent Office**

**Office européen des brevets**

# EUROPÄISCHER RECHERCHENBERICHT

Nummer der Anmeldung

EP 19 20 0482

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | DE 22 42 626 A1 (BAYER AG) 14. März 1974 (1974-03-14) * Anspruch 1 * * Seite 6, Absatz 3 * * Seite 9, Absatz 2 - Absatz 3 * * Beispiel 1 * ----- | 1,8,9,12 | INV. C07C263/20 C07C265/14 B01J3/00 F04C19/00 |

RECHERCHIERTE SACHGEBIETE (IPC)

C07C
F04C
B01J

Der vorliegende Recherchenbericht wurde für alle Patentansprüche erstellt

| Recherchenort | Abschlußdatum der Recherche | Prüfer |
|---|---|---|
| Den Haag | 27. März 2020 | Seitner, Irmgard |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

...........................................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 19 20 0482

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

27-03-2020

| Im Recherchenbericht angeführtes Patentdokument | Datum der Veröffentlichung | Mitglied(er) der Patentfamilie | Datum der Veröffentlichung |
|---|---|---|---|
| DE 2242626 A1 | 14-03-1974 | AU 5959673 A | 27-02-1975 |
| | | BE 804096 A | 28-02-1974 |
| | | DE 2242626 A1 | 14-03-1974 |
| | | ES 418296 A1 | 01-06-1976 |
| | | FR 2197837 A1 | 29-03-1974 |
| | | GB 1441753 A | 07-07-1976 |
| | | IT 998482 B | 20-01-1976 |
| | | JP S4959096 A | 07-06-1974 |
| | | NL 7311766 A | 04-03-1974 |
| | | US 3954567 A | 04-05-1976 |

Für nähere Einzelheiten zu diesem Anhang : siehe Amtsblatt des Europäischen Patentamts, Nr.12/82

EPO FORM P0461

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 3549504 A
- WO 2017076551 A1
- DE 10260092 A1
- DE 2242626 A1
- DE 10019718 A1
- WO 2006029884 A1

**In der Beschreibung aufgeführte Nicht-Patentliteratur**

- **W. SIEFKEN.** *Liebigs Annalen der Chemie,* 1949, vol. 562, 96